# EUROPEAN PATENT SPECIFICATION

(11) **EP 2 538 917 B1**
(45) Date of publication and mention of the grant of the patent: **16.11.2016**
(21) Application number: 11737358.9
(22) Date of filing: 24.01.2011
(51) Int. Cl.: A61K 8/97, A61Q 7/00, A61Q 19/00, A61Q 19/02, A61Q 19/06, A61Q 19/08, A61K 36/898, A61K 36/185, A61Q 17/04

(54) **COMPOSITION CONTAINING EXTRACT OF VENUS FLYTRAP FOR COSMETIC TREATMENT**
ZUSAMMENSETZUNG MIT EINEM VENUSFLIEGENFALLENEXTRAKT ZUR KOSMETISCHEN ANWENDUNG
COMPOSITION CONTENANT DE L'EXTRAIT DE DIONÉE ATTRAPE-MOUCHES, POUR TRAITEMENT COSMÉTIQUE

(30) Priority: 26.01.2010 SE 1050080
(43) Date of publication of application: 02.01.2013
(73) Proprietor: Quantum Pharmaceuticals Ltd., Hong Kong (CN); Royal Government of Bhutan, Ministry of Agriculture, Thimphu (BT)
(72) Inventor: DAHLGREN, Atti-La, CH-2000 Neuchatel (CH); DAHLGREN, John, CH-2074 Marin-Epagnier (CH); DORJI, Tashi Yangzome, Thimphu (BT); DORJI, Singay, Sherbithang (BT); GYELTSHEN, Sherub, Thimphu (BT)
(74) Representative: Fagerlin, Heléne
(86) International application number: PCT/SE2011/050067
(87) International publication number: WO 2011/093769

(56) References cited:
- EP-A1- 0 211 093
- EP-A2- 0 249 165
- WO-A1-99/42115
- JP-A- 2005 289 872
- KREHER ET AL: "Naphthoquinones from Dionaea muscipula", PHYTOCHEMISTRY, PERGAMON PRESS, GB, vol. 29, no. 2, 1 January 1990 (1990-01-01), pages 605-606, XP022515617, ISSN: 0031-9422
- DATABASE WPI Week 200245 Thomson Scientific, London, GB; AN 2002-420402 XP002721204, & JP 2002 053453 A (NOEVIR KK) 19 February 2002 (2002-02-19)

## Description

The present invention relates to an extract of Dionaea muscipula including all wild types and cultivars and a composition comprising such an extract for use in cosmetic treatment of the skin, especially skin changes due to accelerated or chronological aging e.g. wrinkles, loss of firmness and elasticity, and increased pigmentation. The composition may also contain an extract from Cymbidium erythraeum and other ingredients.

### BACKGROUND OF THE INVENTION

### Field of the Invention

This invention comprising an extract of Dionaea muscipula, relates to obtain new and safe antioxidants, humectants, peptides, enzyme inhibitors such as thyrosinase inhibitors, or cosmetic compositions having ameliorating effects on the skin, especially skin changes due to accelerated or chronological aging e.g. wrinkles, loss of firmness and elasticity, and increased pigmentation, ameliorate dried and chapped skin, improve firmness and elasticity of the skin, improve oily skin and pore size, and be used to prevent and/or treat accelerated skin ageing. Furthermore the invention provides a new solution to treat disturbances in the growth and quality of skin appendages such as sebaceous glands, hair and nails.

The biological ageing process, in particular the ageing process of the skin has genetic as well as environmental causes. The environmental causes comprise the internal physiological environment of the individual organism and external influences. The internal environment is reflected by the physiological homeostasis, hormonal balance, utilisation of nutrients, cellular repair mechanisms and antioxidant status. External environmental ageing factors may be chronic radiation (UV) injury (CRI) from the sunlight, smoking, diet, stress and lifestyle. Many of these external environmental factors have an accelerating influence on the ageing processes through e.g. formation of free radicals. Accelerated skin ageing is mainly caused by external factors, resulting in a skin that appears older than the chronological age of individuals not exposed in the same degree to the causative environmental factors. The use of an extract from Dionaea muscipula has surprisingly shown to counteract the effects of these environmental influences and protect the skin and supporting the organisms own repair mechanisms. The use of Dionaea muscipula extracts to improve the physiological status and the cosmetic appearance of the skin has not been described before.

### Description of Prior Art

Dionaea muscipula is a carnivorous plant 5 - 25 cm tall found in the wild in the United States of America in nutrient poor marsh lands. It can be cultivated in sustainable conditions indoors all over the world.

WO 1999/042115 relates to the use of extracts from plants among others from the family Drosera and especially Dionaea muscipula for the production of a medicament for inhibition of protein kinases. The extracts may be used as antibiotics for the treatment of infections of various micro-organisms.

EP 0 211 093 A1, EP 0 249 165 A2 and WO 99/42115A1 all show extracts of *Dionaea muscipula.* None of these documents mention a cosmetic use of the extract or composition comprising the extract.

JP 2005 289872 A shows the use of an extract from another plant, Cymbidium for cosmetic treatment.

Kreher et al. : "Naphthoquinones from Dionaea muscipula", Phytochemistry, Pergamon Press, GB, vol. 29, no.2, 1 January 1990 (1990-01-01), pages 605-606, XP022515617, ISSN: 0031-9422 discloses the isolation of the known naphthoquinones, plumbagin, droserone and 3-chloroplumbagin and a new naphthoquinone, hydroplumbagin 4-O β-glucopyranoside from a methanolic extract of *Dionaea muscipula.* It also mentions that *Dionaea muscipula* has been used as an anticancer drug and that plumbagin enhances in vitro phagocytosis of human granulocytes. Cosmetic use is not disclosed

Database WPI, Week 200245, Thomson Scientific, London, GB discloses a skin whitening cosmetic which contains extract of plants belonging to another genus, such as *Drosera rotundifolia* or *Drosera spathulata* belonging to the Droseraceae family. *Dionaea muscipula* belonging to the Dionaea genus is not mentioned.

### SUMMARY OF THE INVENTION

The present invention relates to the use of an extract of Dionaea muscipula and a composition comprising such an extract for cosmetic treatment of the skin, especially skin changes due to accelerated or chronological aging e.g. wrinkles, loss of firmness and elasticity, and increased pigmentation. It also regards the use of such an extract for the preparation of a composition for cosmetic treatment. The composition may comprise further substances active against skin changes e.g. wrinkles, loss of firmness and elasticity, and increased pigmentation due to accelerated or chronological aging. Especially it may contain an extract from Cymbidium erythraeum. The composition may also comprise bases, cosmetic pigments, UV filters and/or UV scattering agents.

The scope of the invention also covers a method for cosmetic treatment of the skin, especially skin changes due to accelerated or chronological aging e.g. wrinkles, loss of firmness and elasticity, and increased pigmentation herein a person's skin is treated with a composition comprising an extract from Dionaea muscipula. The composition may also comprise an extract from Cymbidium erythraeum.

### DETAILED DESCRIPTION OF THE INVENTION

According to one aspect, the invention relates to an extract of Dionaea muscipula for use in cosmetic treatment of the skin, especially skin changes due to accelerated or chronological aging e.g. wrinkles, loss of firmness and elasticity, and increased pigmentation, especially melanin pigmentation and age spots. It also relates to a composition comprising an extract of Dionaea muscipula for such a use. Consequently, the invention also regards the use of extract of Dionaea muscipula for the preparation of a composition for cosmetic treatment of the skin, especially skin changes due to accelerated or chronological aging e.g. wrinkles, loss of firmness and elasticity, and increased melanin pigmentation. Furthermore it comprises new solutions to treat skin that has lost its firmness and elasticity e.g. re-shaping problem-skin such as cellulites (Gynoid Lipodystrophy), sagging skin and stretch marks (striae) by inducing a strong 'lifting effect'. The invention also provides a new solution to treat disturbances in the growth and quality of skin appendages such as sebaceous glands, hair and nails.
Further the invention also provides a new solution for the treatment of disorders of the sebaceous glands, e.g. inflammation or blocking of pores, oily skin and Acne especially Acne vulgarise. Further the invention provides a new solution for thinning of hair and support hair growth in both males and females. Further the invention provides for a new solution to thin and brittle nails.

According to a further aspect the composition may further comprise at least one other substance active against accelerated or chronological aging changes of the skin such as wrinkles, loss of firmness and elasticity, and increased melanin pigmentation. This may be chosen from any substance active against accelerated or chronological aging changes of the skin such as wrinkles, loss of firmness and elasticity, and increased melanin pigmentation According to one aspect the at least one other substance is chosen from an extract of Cymbidium erythraeum. The composition may comprise one or more extracts of Dionaea muscipula and/or Cymbidium erythraeum.

The extract and the composition according to the invention may be used against accelerated or chronological aging changes of the skin such as wrinkles, loss of firmness and elasticity, and increased melanin pigmentation caused by diseases, inflammation, environmental agents and/or ageing.

Thus, the wrinkles in the skin may be caused by, eczematous changes of the skin in general that often are observed in the absence of skin diseases but also occurs as a consequence of many specific dermatological diseases including skin atopy that often accompanies nasal allergy and asthma, psoriasis, lichen planus, bullous pemphigoid, pemphigus, and dermatitis. Skin diseases may also arise as a consequence of gene defects the patient lacking specific enzymes.

### Extract derived from Dionaea muscipula

A therapeutic extract may be derived from Dionaea muscipula, found in the wild or in cultivation around the world for example, the United States of America, The Kingdom of Bhutan, India, The Peoples Republic of China, South East Asia, Tropical or subtropical regions of the world, indoor or outdoor cultivation in all countries and territories of the world.

According to one embodiment of the present invention the Dionaea muscipula extract may be obtained from a process comprising the following non-limiting procedures using the whole plant, or any part thereof e.g. the leaves, flowers, seeds, cells, stem cells and or root of the plant including tissues, cells and or stem cells obtained from the plant or cultivated in vitro.
1. Fresh juice extract obtained by physical pressure to the plant material.
2. Freeze drying
3. Frementation ( enzymatic, bacterial and or fungal)
4. Water extraction
5. Extraction using super-critical CO2 (Carbon dioxide)
6. Extraction using a suitable solvent (e.g. alcohols, ethanol, isopropyl alcohol, Acetyl acetate, Acetone, etc)

According to one embodiment of the invention the extraction procedure would be:
(a) placing Dionaea muscipula material in a suitable solvent, preferably 96% ethanol in water to achieve extraction of the plant material;
(b) separating the liquid and solid contents;
(c) recovering a first fraction from the liquid contents by filtration of the solvent present in the liquid contents;
(d) placing the solid contents in an organic solvent selected from the group of solvents consisting of but not limited to: acetone, carbon dioxide, isopropanol or t-butanol and esters of acetic acid, preferably ethyl acetate to achieve extraction of the remaining fraction from the plant material;
(e) separating the liquid and solid contents;
(f) recovering a second fraction by filtration of the solvent from the liquid contents; and
(g) recovering the solid contents.

### Extract of Cymbidium erythraeum

Cymbidium erythraeum is an epiphytic plant 25- 75 cm tall found in The Kingdom of Bhutan and other regions of the Himalayas in open mossy evergreen oak forests at an altitude of 1660-2330 m.

The Cymbidium erythraeum extract may be obtained from a process comprising the following non-limiting procedures using the whole plant, or any part thereof e.g. the leaves, flowers, seeds, cells, stem cells and or root of the plant including tissues, cells and or stem cells obtained from the plant or cultivated in vitro.

In one embodiment of the present invention the Cymbidium erythraeum extract may be obtained from a process comprising the steps of:
(a) placing Cymbidium erythraeum material in a suitable solvent, preferably 70% ethanol in water to achieve extraction of the plant material;
(b) separating the liquid and solid contents;
(c) recovering a first fraction from the liquid contents by filtration of the solvent present in the liquid contents;
(d) placing the solid contents in an organic solvent selected from the group of solvents consisting of but not limited to: acetone, carbon dioxide, isopropanol or t-butanol and esters of acetic acid, preferably ethyl acetate to achieve extraction of the remaining fraction from the plant material;
(e) separating the liquid and solid contents;
(f) recovering a second fraction by filtration of the solvent from the liquid contents; and
(g) recovering the solid contents.

In case of fresh plant juice the extract is calculated as % by weight juice obtained from fresh plant material, e.g. 10 kg of fresh plant material is presses and gives 1 kg of 100% juice.

Extract from dry plant material is calculated as the amount of plant material per quantity of extraction medium. e.g. 1 kg of dry plant material extracted in 10 Kg of 96% ethanol gives a 10% extract.

Fresh juice and/or extract is used in a final concentration between 0.001% - 100%, preferably 0.1% - 20% and most preferred 1%- 10%, e.g. 0,5%-10%, 1%-5% in the composition based in the weight of the composition.

### Dosage

For the treatment of a skin condition with the invention, 2 to 4 applications per day on the skin area are recommended depending on the skin disorder and the severity of the disorder.

### Other active ingredients

Apart from extracts from Dionaea muscipula and Cymbidium erythraeum the composition of the invention may contain but not limited to bioactive carbohydrates, lipids, proteins, peptides, oligo-elements, enzymes, plant tissue factors, enzyme inhibitors, flavonoids, polyphenols and xanthophylls.

Other active ingredients and plant extracts may be added such as but not limited to: ascorbic acid, Tremella Fuciformis, Edelweiss (Leontopodium Spp), Snowflake (Leucojum Spp), Morus alba, Polygonum cuspidatum, Cordyceps Spp, Kojic Acid, Coffeine, Capiscum Spp, Nicotine amide and Mimosa pudica sensitiva may also be used in combination with the invention.

### UV filters.

The composition may also further comprise at least one UV filter and/or UV scattering agent.

As sunshine protecting compounds both UV filters and UV scattering agents may be used. The UV filters may be of UVA and UVB type. Both organic and inorganic substances may be used as UV filters. The ultraviolet shielding agent using organic compounds as an effective component prevents transmission of the ultraviolet light by characteristic absorption of ultraviolet light by the component. For example, an ultraviolet absorbent composition comprising substituted N,N'-bis-aromatic formamidines is known (Japanese Patent Examined Publication No. 61-09993).

On the other hand, the ultraviolet shielding agent using an inorganic compound contains inorganic fine particles in its composition and prevents transmission of the ultraviolet light by the absorbing ability and the scattering ability of the composition to the ultraviolet light.

Inorganic UV filters may be flaky glass of a titania-silica glass (Japanese Patent Laid-Open No. 6-116119); composite fine particles comprising ultrafine particles dispersed in and supported by the solid material e.g. a composite powder in which a fine particle powder such as TiO.sub.2 is uniformly dispersed in plate particles of metal oxides such as SiO.sub.2 (Japanese Patent Laid-Open No. 1-143821); and composite particles in which a zirconium oxide powder or an aluminum oxide powder is carried on a surface of the matrix particles comprising such materials as nylon resins, silicone resins, and silicon oxide, and a titanium oxide powder or a zinc oxide powder is dispersed in an inner portion of the matrix particles (Japanese Patent Laid-Open No. 2-49717); metal compounds-containing porous silica beads (Japanese Patent Laid-Open No. 4-65312): glass powder comprising metal oxides, such as SiO.sub.2, Al.sub.2 O.sub.3, B.sub.2 O.sub.3, BaO, SrO, ZnO, and MgO, and metal fluorides (Japanese Patent Laid-Open No. 4-85346)

In order to improve dispensability, a surface of the ultrafine particles may be coated with other materials. For example, skin cosmetics comprising an oily cosmetic base material and a hydrophobic titanium oxide powder may be used (Japanese Patent Examined Publication No. 59-15885).

### Pigments.

According to one aspect the composition further comprises at least one cosmetic pigment. The pigments may be chosen from various titanic oxides e.g. TiO₂, zinc oxides e.g. ZnO, ferric and ferrous oxides and mica e.g. KAl₂(Al Si₃O₁₀)(OH)₂, KMg₃(AlSi₃O₁₀)(OH)₂

### Bases

The extract may be applied directly to the skin or in a suitable base. Thus, the composition may further comprise at least one base such as an excipient, diluent and vehicle, e.g. lotion, ointment, cream, gel or milk.

With base we understand any excipient or vehicle that is commonly used in formulations for skin treatment such as lotions, ointments, creams, gels etc. The composition for skin treatment according to the invention may be any such formula used in the art for skin treatments.

Suitable topical vehicles for use according to the invention are well known in the cosmetic and pharmaceutical arts, and include such vehicles (or vehicle components) as water; organic solvents such as alcohols (particularly lower alcohols readily capable of evaporating from the skin such as ethanol), glycols (such as glycerol), aliphatic alcohols (such as lanolin); mixtures of water and organic solvents (such as water and alcohol), and mixtures of organic solvents such as alcohol and glycerol (optionally also with water); lipid-based materials such as fatty acids, acylglycerols (including oils, such as mineral oil, and fats of natural or synthetic origin), phosphoglycerides, sphingolipids, liposomes and waxes; protein-based materials such as collagen and gelatin; silicone-based materials (both non-volatile and volatile) such as cyclomethicone, demethiconol and dimethicone copolyol (Dow Corning); hydrocarbon-based materials such as petrolatum and squalene; anionic, cationic and amphoteric surfactants and soaps; sustained-release vehicles such as microsponges and polymer matrices; stabilising and suspending agents; emulsifying agents; and other vehicles and vehicle components that are suitable for administration to the skin, as well as mixtures of topical vehicle components as identified above or otherwise known to the art. The vehicle may further include components adapted to improve the stability or effectiveness of the applied formulation, such as preservatives, skin penetration enhancers, sustained release materials, and the like.

Examples of such vehicles and vehicle components are well known in the art and are described in such reference works as Martindale-The Extra Pharmacopoeia (Pharmaceutical Press, London 1993) and Martin (ed.), Remington's Pharmaceutical Sciences.

The choice of a suitable vehicle will depend on the particular physical form and mode of delivery that the formulation is to achieve. Examples of suitable forms include liquids as well as suspensions, emulsions and the like; solids and semisolids such as gels, foams, pastes, creams, ointments, "sticks"; formulations containing liposomes or other.

The base may also comprise polymeres or substances that act as water swelling agents and/or rheology modifier and chelating agents, such as Carbopol®, Carbomer® and tetrasodium EDTA.

The composition may comprise between 10 and 90 weight % of at least one base. e.g. about 50% to about 90%, preferably from about 70% to about 85% and a system as defined above in an amount between about 10% and about 90%, e.g. from about 10% to about 50%, preferably from about 15% to about 30%.

### New composition

The invention also regards a composition comprising an extract of Dionaea muscipula and an extract of Cymbidium erythraeum.

### Examples of formulations:

### Formulation 1

Gel composition containing Dionaea Muscipula as active ingredient:
Dionaea Muscipula 0.500 g
Carbopol 980 NF 0.250 g
Triethanolamine 0.200 g
Hydroxypropyl-methylcellulose 1.000g
Purified water ad 50.000 g

According to the recipe of the above example the gel was prepared in a batch size of 7 kg with a Brogtech apparatus (Brogtech apparatus: Brogtec Mischtechnik GmbH, Gewerbestrasse 14, D-79618 Rheinfelden, Germany) suitable for the preparation of ointments.

### Method of preparation of the gel base:

Purified water (6000 g) is poured into the Brogtech apparatus and the temperature is set at 25°C. In an anchor mixer in position 4, hydroxypropyl-methylcellulose (140.0 g) is added stepwise to the mixture and it is stirred at the same revs/minute speed until total dissolution of the ointment base (approximately 1.5 hours). After dissolution Carbopol 980 NF (35.0 g) is added to the reaction mixture and it is stirred for 4 hours. The mixture is neutralized with a solution of triethanolamine (28.0 g) and purified water (100.0 g) and stirring is continued until the mixture has gel consistency.

Method of preparation of the end product (drug-gel composition): To the gel base prepared the Dionaea Muscipula extract is added stepwise, and the gel is completed to 7.00 kg with purified water. The obtained gel is homogenized for 5 minutes in the built-in homogenizer of the Brogtech apparatus at 1200 revs/min, at the maximum diameter of the slits (1.5 mm).

### Formulation 2

### Anti ageing cream.

Ingredient % (1) Water 67.7 (2) Dicetyl Phosphate (and) Ceteth-10 Phosphate 6.0 (3) Glyceryl Stearate (and) PEG-100 Stearate 4.0 (4) Phenoxyethanol 0.6 (5) Chlorphenesin 0.4 (60) Titanium Dioxide 0.2 (7) Sodium Hydroxide 0.5 (8) Magnolol 0.2 (9) Dionaea muscipula 1.0 (10) Cetyl Dimethicone 1.5(11) Ergosterol gluconate 1.5 (12) Shea butter 2.0 (13) Tremella fusciformis 1.0 (15) Cymbidium erythraeum 1.0 (16) Cordyceps sinensis 0.1 (17) Zinc Hydroxycitrate 3.1 (18) 2,4-Dihydroxy Acetophenone (Resacetophenone) 1.1 (19) Paeonol 1.5 (20) Carnosine 0.1 (21) Cyclomethicone, Dimethicone Crosspolymer 2.0 (22) Mimosa pudica extract 0.5 (23) Polysorbate-20 2.0 (24) Sepigel-305 2.0 (Sepigel-350 (polyacrylamide, C 13 C 14 isoparaffin and laureth-7):SEPPIC GmbH, ABC Tower Köln, Ettore - Bugatti - Str.6-14, 51149 Köln-Porz, GERMANY.)

### Procedure.

Mix (1) to (13) and heat at 70 to 80 C till homogenous. Cool to 40 to 50 C. Premix (14) to (16) and add to batch with mixing. Add all other ingredients and mix. Cool to room temperature. An off-white cream is obtained.

### Formulation 3

### Acne Cream with Sebum Reduction.

Ingredient % (1) Water 67.3 (2) Dicetyl Phosphate (and) Ceteth-10 Phosphate 5.0 (3) Glyceryl Stearate (and) PEG-100 Stearate 4.0 (4) Phenoxyethanol 0.7 (5) Chlorphenesin 0.3 (60) Titanium Dioxide 0.2 (7) Sodium Hydroxide 0.5 (8) Magnolol 0.2 (9) Dionaea muscipula 0.5 (10) Cetyl Dimethicone 1.5 (11) Tetrahydrocurcuminoids 0.5 (12) Shea butter 2.0 (13) Ximenia oil 1.0 (15) Ascorbic acid 4.0 (16) Niacinamide Hydroxycitrate 2.2 (17) 2,4-Dihydroxy Acetophenone (Resacetophenone) 1.1 (18) Hecogenin 1.5 (19) Carnosine 0.1 (20) Cyclomethicone, Dimethicone Crosspolymer 2.0 (21) Morus alba 0.5 (22) Pyridoxine Salicylate 0.9 (23) Polysorbate-20 2.0 (24) Sepigel-305 2.0.

### Procedure.

Mix (1) to (13) and heat at 70 to 80 C till homogenous. Cool to 40 to 50 C. Premix (14) to (16) and add to batch with mixing. Add all other ingredients and mix. Cool to room temperature. An off-white cream is obtained.

### Formulation 4

### Anti-aging Gel

Ingredients % (1) Triethyl Citrate 63.95 (2) Ethylenediamine/Hydrogenated Dimer Dilinoleate Copolymer Bis-Di-C14-18 Alkyl Amide 10.0 (3) Jojba oil 0.1 (4) Mimosa pudica 0.25 (5) Dionaea muscipula extract 2.0 (6) Protodioscin 0.5 (7) Cymbidium erythraeum 0.2 (8) Zeolite 20.0 (9) Fragrance 1.0 (10) Ascorbic acid 2.0

### Procedure.

Mix (1) and (2) and heat at 80 to 90 C till clear. Cool to 40 to 50 C and add all other ingredients and mix. Cool to room temperature. A white gel-like product is obtained.

### Formulation 5

### Anti-Wrinkle, Sebum Reduction Transparent Gel.

Ingredients % (1) C12-15 Alkyl Benzoate 96.75 (2) Dibutyl Lauroyl Glutamide 1.0 (3) Jojoba Oil 0.1 (4) Tremella fuciformis 0.25 (5) Leontopodium extract 0.2 (6) Paeonol 0.5 (7) Tetrahydrocurcuminoids 0.2 (8) Dionaea muscipula 1.0.

Procedure. Mix (1) and (2) and heat at 95 to 110 C till clear. Cool to 40 to 50 C and add all other ingredients and mix. Cool to room temperature. A transparent gel-like product is obtained.

### Formulation 6

### Anti cellulite- reshaping gel

Ingredients % (1) Triethyl Citrate 63.75 (2) Ethylenediamine/Hydrogenated Dimer Dilinoleate Copolymer Bis-Di-C14-18 Alkyl Amide 10.0 (3) Jojba oil 0.1 (4) Nicotinamide 0.25 (5) Dionaea muscipula extract 2.2 (6) Protodioscin 0.5 (7) Cymbidium erythraeum 0.2 (8) Zeolite 19.5 (9) Fragrance 1.0 (10) Coffein 2.0 (11) Capsicum 0.5

### Procedure.

Mix (1) and (2) and heat at 80 to 90 C till clear. Cool to 40 to 50 C and add all other ingredients and mix. Cool to room temperature. A white gel-like product is obtained.

### Formulation 7

### Hair tincture

Ingredients % (1) Dionaea muscipula 10% in Ethanol 92.0 (2) Polysorbatum 80 5.0 (3) Cymbidium erythaeum 2.0 (4) Coffein 1.0

### Procedure

Add all ingredients and mix at 50° C. Cool to room temperature. A liquid is obtained

For use as a regenerating agent for hair growth, approximately 10 to 50 drops of this hair tincture are used daily, rubbed into the scalp and on the hair.

### Formulation 8

### Nail cream

Ingredients % (1)Collagen 3.0 (2) Dionaea muscipula 5.0 (3) Glycerol 2.0 (4) Dipropylene glycol 3.0
(5) Polyoxyethylene isocetyl ether 1.0 (6) Ethanol 8.0 (7) Fragrance 0.5 (8) Purified water ad to 100

### Procedure

Add all ingredients and mix at 60° C. Cool to room temperature. A whitish cream is obtained.

The nail cream is applied to the nails twice daily

### EXAMPLES

The present invention will be more readily understood by referring to the following examples which are given to illustrate the invention rather than to limit its scope.

### Example 1.

An adult Caucasian female (36 years) is regularly working out-doors, and routinely exposed to UV light from the sun. Without protection she easily burns her skin. The redness and inflammation of the skin resulted in a great discomfort. She started to apply Dionaea Extract in a gel formulation according to the invention (Formulation 1). After a few minutes the pain was relieved and after 20 minutes the redness was gone. Due to regular exposure to the sunlight she has freckles and pigment spots on sun exposed skin areas. After using Dionaea extract according to the invention for 4 weeks the pigment spots were gradually reduced and disappeared. After some time she interrupted the use of the invention and was again working out-doors. The pigments pots started to reappear but were not as numerous as before. When she again started using the invention the pigment spots disappeared. Thus it can be seen from example one that the use of Dionaea extract can alleviate skin damage and pigmentation changes induced by UV radiation.

### Example 2.

A 45 year old Asian woman started to notice a gradual overall loss of skin elasticity and firmness of her skin, in particular in the face and bust. Around the eyes fine lines 'crow's feet' were visible. She started to apply Dionaea extract in a cosmetic formulation (Formulation 2) on her face and bust two times per day. Already after a few applications she notices a strong lifting effect. During the cause of 1 month she noticed a gradual improvement of the skin quality, the skin looked more youthful and radiant. And after 3 months her skin felt more firm and she noticed that the fine lines around her eyes were markedly diminished.

### Example 3.

A 69 year old Japanese man, who had spend a lot of time in the sun playing sports (golf), showed signs of extended accelerated skin aging especially in the back if his hands and face with prominent wrinkling and numerous age spots. His skin was investigated by a skin scientist who objectively could determine an extensive micro scar damage and reduction in collagen content and elasticity of the skin.

He started using a cosmetic composition according to the invention (Formulation 4). After 3 months another objective assessment was done by the same skin scientist. This showed an improved regeneration of collagen and improved elasticity of the treated skin areas. The age spots were significantly reduced. His own impression was a marked improvement of his skin condition.

After 3 months he interrupted the use of the invention. One month after the interruption he was reassessed by the same skin scientist. The results showed a small but significant reduction of the previous improvements, proving that a continuous use of the invention is necessary to maintain the positive effects obtained by the treatment.

### Example 4.

A 23 year old Asian woman was suffering since her mid-teens from oily and Acne prone skin on her face and shoulders. Her skin pores were enlarged and often blocked by comedones (so called black heads and white heads). She started to use a gel formulation ( Formulation 5) according to the invention. After two weeks of use the acne started to clear up, the skin was markedly less oily, and the comedones started to diminish. After 4 weeks the skin had cleared up, pores were smaller and previous acne scars were less prominent.

### Example 5

An Asian woman (38 years) spending a lot of time out doors in south-east Asia, were suffering from irregular melanin skin coloration caused by repeated strong exposure to sun radiation. She consulted a skin specialist who applied the invention (Formulation 3) to her skin. Already after two weeks usage the skin tone become lighter measured by an objective skin tone scale and the overall skin tone become more even.

### Example 6

A 36 year old Caucasian woman mother of two children, suffered from so called orange peel skin or cellulites over both buttocks and hips. Furthermore she suffered from stretch marks over her stomach after two pregnancies. She started applying the invention (formulation 6) on the affected areas for 8 weeks. After this period a remarkable improvement of the cellulites and the stretch marks could be observed. The skin was firmer, smoother, more elastic and had a more youthful appearance.

## Claims

1. Use of a composition comprising an extract of Dionaea muscipula in cosmetic treatment of the skin, especially skin changes due to accelerated or chronological aging e.g.wrinkles, loss of firmness and elasticity, and increased pigmentation.

2. The use according to claim 1 **characterised in that** the composition comprises at least one other substance active against skin changes due to accelerated or chronological aging e.g. wrinkles, loss of firmness and elasticity, and increased pigmentation.

3. Use of the composition according to any of claims1 and 2, **characterised in that** the at least one other substance active against skin changes due to accelerated or chronological aging e.g. wrinkles, loss of firmness and elasticity, and increased pigmentation is an extract from Cymbidium erythraeum.

4. Use of the composition according to any of claims 1-3, **characterised in that** the composition further comprises at least one base such as an excipient, diluent and vehicle, e.g. lotion, ointment, cream, gel or milk.

5. Use of the composition according to claim 4, **characterised in that** the composition comprises between 10 and 90 weight % of at least one base.

6. Use of the composition according to any of claims 1-5, **characterised in that** the composition further comprises at least one agent chosen from bioactive carbohydrates, lipids, proteins, peptides, oligo- elements, enzymes, plant tissue factors, enzyme inhibitors, flavonoids, polyphenols and xanthophylls, ascorbic acid, Tremella Fuciformis, ascorbic acid, Edelweiss (Leontopodium Spp), Snowflake (Leucojum Spp), Morus alba, Polygonum cuspidatum, Cordyceps Spp, Kojic Acid, Coffeine, Capiscum Spp, Nicotinamide and Mimosa pudica sensitiva may also be used in combination with the invention.

7. Use of the composition according to any of claims 1-6, **characterised in that** the composition further comprises at least one UV filter and/or UV scattering agent and/or at least one cosmetic pigment.

## Patentansprüche

1. Verwendung einer Zusammensetzung, die einen Extrakt von Dionaea muscipula enthält, bei der kosmetischen Behandlung der Haut, insbesondere der Hautveränderungen, die durch das beschleunigte oder chronologische Altern verursacht sind, wie z.B. Falten, Verlust von Festigkeit und Spannkraft und erhöhter Pigmentierung.

2. Verwendung nach Anspruch 1, **dadurch gekennzeichnet, dass** die Zusammensetzung mindestens eine andere Substanz umfasst, die aktiv gegen die Hautveränderungen ist, die durch das beschleunigte oder chronologische Altern verursacht sind, wie z.B. Falten, Verlust von Festigkeit und Spannkraft und erhöhter Pigmentierung.

3. Verwendung der Zusammensetzung nach einem der Ansprüche 1 und 2, **dadurch gekennzeichnet, dass** die mindestens eine andere Substanz, die aktiv gegen die Hautveränderungen ist, die durch das beschleunigte oder chronologische Altern verursacht sind, wie z.B. Falten, Verlust von Festigkeit und Spannkraft und erhöhter Pigmentierung, ein Extrakt von Cymbidium erythraeum ist.

4. Verwendung der Zusammensetzung nach einem der Ansprüche 1-3, **dadurch gekennzeichnet, dass** die Zusammensetzung ferner mindestens eine Base umfasst, wie z.B. einen Hilfsstoff, Verdünnungsmittel und Träger, z.B. Lotion, Salbe, Creme, Gel oder Milch.

5. Verwendung der Zusammensetzung nach Anspruch 4, **dadurch gekennzeichnet, dass** die Zusammensetzung zwischen 10 und 90 Gewichts-% mindestens einer Base umfasst.

6. Verwendung der Zusammensetzung nach einem der Ansprüche 1-5, **dadurch gekennzeichnet, dass** die Zusammensetzung ferner mindestens ein Mittel umfasst, ausgewählt aus bioaktive Kohlenhydrate, Lipide, Proteine, Peptide, Oligo-Elemente, Enzyme, Pflanzengewebefaktoren, Enzyminhibitoren, Flavonoide, Polyphenole und Xanthophylle, Ascorbinsäure, Silberohr, Ascorbinsäure, Edelweiss (Leontopodium spp), Knotenblume (Leucojum spp), Morus alba, Polygonum cuspidatum, Cordyceps spp, Kojisäure, Koffein, Capsicum spp, Nicotinamid und Mimosa pudica sensitiva können in Kombination mit der Erfindung auch verwendet werden.

7. Verwendung der Zusammensetzung nach einem der Ansprüche 1-6, **dadurch gekennzeichnet, dass** die Zusammensetzung ferner mindestens ein UV-Filter und/oder UV-Streumittel und/oder mindestens ein kosmetisches Pigment umfasst.

## Revendications

1. Utilisation d'une composition comprenant de l'extrait de Dionaea muscipula dans le traitement cosmétique de la peau, notamment des changements de la peau dus à un vieillissement accéléré ou chronologique, par exemple, les rides, la perte de fermeté et d'élasticité, et l'augmentation de la pigmentation.

2. Utilisation selon la revendication 1, **caractérisée en ce que** la composition comprend au moins une autre substance active contre les changements de la peau dus à un vieillissement accéléré ou chronologique, par exemple, les rides, la perte de fermeté et d'élasticité, et l'augmentation de la pigmentation.

3. Utilisation de la composition selon l'une quelconque des revendications 1 et 2, **caractérisée en ce qu'**au moins une autre substance active contre les changements de la peau dus à un vieillissement accéléré ou chronologique, par exemple, les rides, la perte de fermeté et d'élasticité, et l'augmentation de la pigmentation, est un extrait de Cymbidium erythraeum.

4. Utilisation de la composition selon l'une quelconque des revendications 1-3, **caractérisée en ce que** la composition comprend en outre au moins une base, tel que par exemple un excipient, diluant et véhicule, par exemple, lotion, pommade, crème, gel ou lait.

5. Utilisation de la composition selon la revendication 4, **caractérisée en ce que** la composition comprend entre 10 et 90% poids d'au moins une base.

6. Utilisation de la composition selon l'une quelconque des revendications 1-5, **caractérisée en ce que** la composition comprend en outre au moins un agent choisi parmi les glucides, lipides, protéines, peptides, oligo-éléments, enzymes, facteurs de tissu végétal, inhibiteurs enzymatiques, flavonoïdes, polyphénols et xanthophylles, acide ascorbique, Tremella fuciformis, acide ascorbique, edelweiss (Leontopodium spp), nivéole (Leucojum spp), Morus alba, Polygonum cuspidatum, Cordyceps spp, acide kojique, caféine, Capsicum spp, nicotinamide et mimosa pudique peuvent aussi être utilisés en conjonction avec l'invention.

7. Utilisation de la composition selon l'une quelconque des revendications 1-6, **caractérisée en ce que** la composition comprend en outre au moins un filtre UV et/ou un agent de diffusion UV et/ou au moins un pigment cosmétique.
